# EUROPÄISCHE PATENTSCHRIFT

(11) **EP 2 111 797 B1**
(45) Veröffentlichungstag und Bekanntmachung des Hinweises auf die Patenterteilung: **14.09.2016**
(21) Anmeldenummer: 09155013.7
(22) Anmeldetag: 12.03.2009
(51) Int. Cl.: A61B 6/00, A61B 6/04

(54) **Verfahren zur Erzeugung eines Bildes mit einem Mammographiegerät**
Method for creating an image with a mammography device
Procédé de production d'une image à l'aide d'un appareil de mammographie

(30) Priorität: 24.04.2008 DE 102008020670
(43) Veröffentlichungstag der Anmeldung: 28.10.2009
(73) Patentinhaber: Siemens Healthcare GmbH, 91052 Erlangen (DE)
(72) Erfinder: Hoheisel, Martin, 91056, Erlangen (DE)

(56) Entgegenhaltungen:
- EP-A- 1 864 612
- US-A- 6 049 583
- US-A1- 2004 234 113
- US-A1- 2006 098 855
- US-A1- 2007 238 966
- FREDMAN, A.J. ; FROLIK, J.L. ; GARRA, B.S.: "Lung strain profiles using computed tomography elastography" ENGINEERING IN MEDICINE AND BIOLOGY SOCIETY, 2004. IEMBS '04. 26TH ANNUAL INTERNATIONAL CONFERENCE OF THE IEEE, 1. September 2004 (2004-09-01), - 5. September 2004 (2004-09-05) Seiten 1545-1548, XP002533075

## Beschreibung

Die Erfindung betrifft ein Verfahren, und Mammographiegerät nach Anspruch 1 oder 7, zur Erzeugung eines Bildes mit einem Mammographiegerät umfassend eine Strahlungsquelle, einen digitalen Strahlungsdetektor, eine Auflageplatte und eine Kompressionsplatte, zwischen denen die Mamma, die über die Kompressionsplatte komprimiert wird, aufgenommen ist.

Das erfolgreichste Verfahren zur Untersuchung der weiblichen Brust zur Ermittlung etwaiger verdächtiger Läsionen ist die Mammographie, sei es im Rahmen eines verdachtsunabhängigen Screenings oder bei bereits gegebenem Verdacht auf Brustkrebs. Hierzu wird bekanntlich ein Mammographiegerät eingesetzt, umfassend eine Röntgenstrahlungsquelle und einen digitalen Strahlungsdetektor sowie eine Auflageplatte und eine Kompressionsplatte, zwischen welchen Platten die Brust aufgenommen wird, die über die vertikal verstellbare Kompressionsplatte komprimiert wird. Sie befindet sich dann zwischen der Strahlungsquelle und dem digitalen Strahlungsdetektor, so dass hierüber ein Strahlungsbild der Brust aufgenommen werden kann. Dabei ist die Sensitivität des Verfahrens hoch. Üblicherweise erfolgt, wenn ein verdächtiger Bereich im aufgenommenen Mammographiebild erkannt wird, eine weitere Abklärung durch Entnahme einer Biopsie, es erfolgt also ein chirurgischer Eingriff. Dies ist jedoch für die Patientin eine problematische Belastung, ferner sind beachtliche Kosten damit verbunden. Um deshalb die Spezifität des Verfahrens respektive der vorgenommenen Diagnose anhand des Mammographiebildes zu verbessern und eine gutartige Läsion von einer malignen Läsion zu unterscheiden, werden in Zweifelsfällen häufig verschiedene zusätzliche Verfahren eingesetzt wie beispielsweise Ultraschalluntersuchungen, eine optische Durchleuchtung oder eine Magnetresonanzuntersuchung. Diese Verfahren haben jedoch ebenfalls Nachteile. So sind Ultraschalluntersuchungen zeitaufwändig und müssen vom Arzt selbst durchgeführt werden. Die optische Durchleuchtung ist als Verfahren noch wenig etabliert und nicht ausreichend spezifisch, während Magnetresonanzuntersuchungen sehr kosten- und zeitaufwändig sind.

Der Erfindung liegt damit das Problem zugrunde, ein Verfahren zur Erzeugung eines Mammographiebildes anzugeben, das gegenüber einer einfachen Mammographiebildaufnahme einen höheren Grad an Spezifität ermöglicht.

Zur Lösung dieses Problems ist ein Verfahren der eingangs genannten Art mit folgenden Schritten vorgesehen:
- Aufnahme eines die Mamma abbildenden ersten Bilddatensatzes mit einem ersten Kompressionsgrad der Mamma,
- Einstellung eines zweiten Kompressionsgrads der Mamma und Aufnahme eines die Mamma abbildenden zweiten Bilddatensatzes und
- rechnerische Verknüpfung des ersten und des zweiten Bilddatensatzes zur Erzeugung des Bildes.

Das offenbarte Verfahren integriert eine Elastographie in das Verfahren der Mammographie. Beim offenbarten Verfahren werden zwei separate Bilddatensätze respektive Bilder der komprimierten Mamma aufgenommen, wobei die Mamma jedoch jeweils unterschiedlich stark komprimiert, mithin verformt ist. Infolge der Veränderung des Kompressionsgrads vom ersten Bild zum zweiten Bild kommt es zwangsläufig zu einer geometrischen Gewebeveränderung, das heißt, dass sich das Gewebe, ausgehend von der gegebenen Geometrie respektive Gewebeverteilung während der ersten Bildaufnahme, lagemäßig verändert, es verschiebt sich kompressionsänderungsbedingt im Raum. Nach Einstellung des zweiten Kompressionsgrads wird nun ein zweites Bild aufgenommen. Diese beiden Bilder werden anschließend rechnerisch miteinander verknüpft, um ein Endbild zu erzeugen, das dann, gegebenenfalls auch zusammen mit den beiden aufgenommenen Einzelbildern, an einen Monitor ausgegeben werden kann.

Der Elastographie liegt grundsätzlich die Erkenntnis zugrunde, dass das elastische Verhalten verschiedener Gewebestrukturen unterschiedlich ist. Weiches, gesundes Gewebe bewegt sich bei Ausübung eines Drucks auf das Gewebe anders als hartes, krankes Gewebe. Dem offenbarten Verfahren liegt nun die Überlegung zugrunde, dass sich eine gutartige Läsion elastographisch gesehen anders verhält, mithin also bei Druckbeaufschlagung im Raum bewegt, als eine maligne Läsion, also ein canzerogenes Gewebe. Solange die Mamma also ein im Wesentlichen homogenes, plastisches Gewebe darstellt, erfolgt die Verformung im Wesentlichen gleichförmig und vorhandene Gewebeteile, Drüsen, Lipome etc verschieben sich im Wesentlichen gleichmäßig und homogen im Raum. Maligne Läsionen sind dagegen wesentlich geringer verformbar, so dass sie sich in dem sie umgebenden homogenen Gewebe auf abweichende Weise verschieben und verformen. Diesen aus der unterschiedlichen Elastizität resultierenden Unterschied nutzt nun das offenbarte Verfahren, indem die beiden zu verknüpfenden Bilder beziehungsweise Bilddatensätze bei unterschiedlichen Mammakompressionen aufgenommen werden und rechnerisch miteinander verknüpft werden, um das auszugebende, diagnostisch auszuwertende Bild zu erzeugen. In diesem lassen sich aufgrund der rechnerischen Verknüpfung sodann aus dem unterschiedlichen Elastizitätsverhalten, also aus der Elastographie resultierende Bildinformationen entnehmen und diagnostisch auswerten, das heißt, dass sich eine etwaige maligne Läsion wesentlich genauer erkennen lässt, als anhand nur einer einzelnen Mammographie-Durchleuchtungsaufnahme.

Das offenbarte Verfahren kombiniert also die hohe Sensitivität des Röntgen-Durchleuchtungsverfahrens der Mammographie mit den Vorteilen einer Elastographie, wodurch sich die Spezifität des Verfahrens eklatant verbessert, nachdem nicht nur mammographische Informationen, sondern auch elastographische Informationen in das auszugebende, rechnerisch erstellte Endbild einfließen.

Nach einer besonders zweckmäßigen Erfindungsausgestaltung ist vorgesehen, dass einer der beiden Bilddatensätze vor der Verknüpfung unter Berücksichtigung beziehungsweise Annahme eines homogenen elastischen Verhaltens der Mamma derart transformiert wird, dass die aus der Veränderung des Kompressionsgrads resultierende elastische Veränderung der abgebildeten Mamma kompensiert wird. Es erfolgt hier also eine rechnerische Kompensation der mittleren, gleichförmigen Verformung der Mamma, die aus der Veränderung des Kompressionsgrads erfolgt. Über diese Rückrechnung wird quasi z.B. das zweite aufgenommene Bild beziehungsweise der zweite aufgenommene Bilddatensatz so transformiert, dass dem transformierten Bild näherungsweise der gleiche Kompressionsgrad zugrunde liegt, wie dem zuerst aufgenommenen Bild beziehungsweise Bilddatensatz. Im Ergebnis ist die summarische Ausdehnung der Mamma im transformierten zweiten Bilddatensatz beziehungsweise zweiten Bild gleich der Ausdehnung der Mamma im unbearbeiteten ersten Bild beziehungsweise ersten Bilddatensatz, beide sind also im Endeffekt gleich groß. Diese Rückrechnung geht wie bereits einleitend beschrieben von einem im Wesentlichen homogenen Elastizitätsverhalten des gesamten Mammagewebes aus, um im Rahmen der Rücktransformation die einzelnen Bildpunkte entsprechend rechnerisch unter Berücksichtigung des Änderungsgrads der Kompression zu transformieren. Hier sind unterschiedliche rechnerische Rücktransformationsweisen denkbar, beispielsweise ist es möglich, im Rahmen der rechnerischen Transformation zunächst die Transformation ausgezeichneter Punkte im Bild, beispielsweise der die Mamma begrenzenden Randlinie, auf die entsprechenden Vergleichspunkte im Vergleichsbild, also im ersten aufgenommenen Bild, zu bestimmen, und dann ausgehend von diesem Rücktransformationsgrad den Rücktransformationsgrad für alle anderen Bildpunkte zu ermitteln, etc. Auch eine Rückrechnung des ersten Bilddatensatzes auf den zweiten Bilddatensatz ist denkbar.

Der rechnerischen Verknüpfung liegen dann von der dargestellten Mammagröße her näherungsweise gleiche Bilder zugrunde, was die rechnerische Verknüpfung vereinfacht.

Diese rechnerische Verknüpfung kann beispielsweise durch Bildung eines Differenzbilddatensatzes aus den beiden Bilddatensätzen und Ermittlung eines Differenzbildes aus dem Differenzbilddatensatz erfolgen. Das heißt, dass das vorzugsweise transformierte zweite Bild oder der transformierte zweite Bilddatensatz vom ersten Bild beziehungsweise ersten Bilddatensatz subtrahiert wird. Soweit das Gewebe einheitlich homogen ist, wird das Differenzbild im Idealfall weitestgehend konturlos sein, nachdem sich das gesunde Gewebe homogen verschiebt und infolge der Rückrechnung letztlich das zweite Bild beziehungsweise der zweite Bilddatensatz das gleiche zeigt beziehungsweise beinhaltet wie das erste Bild beziehungsweise der erste Bilddatensatz. Ist allerdings eine oder sind mehrere maligne Läsionen vorhanden, die sich bei der kompressionsbedingten Verformung unterschiedlich verschoben haben, so treten diese im Differenzbetrieb deutlich hervor, da für sie die Annahme eines homogenen elastischen Verhaltens, die der Rücktransformation zugrunde gelegt wurde, nicht zutrifft und es mithin im Bereich dieses Bildabschnitts zu einer tatsächlichen Signaldifferenz innerhalb der betroffenen Pixel kommt. Die reine Subtraktion im Rahmen der Differenzbilddatensatzerzeugung ist nur ein Beispiel, es sind unterschiedlichste Rechenformeln oder Wichtungsformeln im Rahmen der rechnerischen Verknüpfung denkbar. So ist es möglich, im Rahmen der Verknüpfung die beiden Bilddatensätze ungleich zu wichten, um so sicherzustellen, dass die Mamma aus einem Bild, vorzugsweise natürlich dem nicht transformierten Bild, im rechnerisch ermittelten auszugebenden Endbild noch leicht sichtbar bleibt, was die Orientierung für den Arzt erleichtert, wenn er das Bild auswertet. Auch kann dem Bediener die Möglichkeit gegeben werden, beispielsweise durch Grauwertfensterungen oder sonstige Bildverarbeitungstools Parameter für die rechnerische Verknüpfung einzustellen, die die Bildinhalte der einzelnen zu verknüpfenden Bilddatensätze unterschiedlich berücksichtigt beziehungsweise wichtet. Das heißt, dass der Bediener bedarfsabhängige Einstellungen vornehmen kann, wie die rechnerische Verknüpfung nun konkret erfolgen soll, so dass er im Rahmen der Bildverarbeitung die Verarbeitungsparameter so einstellen kann, dass ein für ihn optimales, diagnostisch auswertbares Endbild, beispielsweise ein Differenzbild erzeugt wird.

Wie bereits beschrieben, besteht die Möglichkeit, beispielsweise durch unterschiedliche Wichtung der Bilddatensätze die Verknüpfung so vorzunehmen, dass die Mamma eines Bildes noch leicht sichtbar bleibt. Denkbar ist es aber auch, die Bilddatensätze derart zu verknüpfen, dass lediglich der Verlauf des Randes der in einem der beiden Bilddatensätze, insbesondere in dem nicht transformierten Bilddatensatz, gezeigten Mamma im Bild, gegebenenfalls im Differenzbild, dargestellt wird. Auch dies ermöglicht es dem Arzt, die genaue Lage eines möglichen Befundes bestimmen zu können. Zur Ermittlung des Rands in dem einen Bilddatensatz kann beispielsweise ein Kantendetektionsalgorithmus herangezogen werden.

Im Rahmen der Durchführung des erfindungsgemäßen Verfahrens ist es für eine möglichst zügige Verfahrensabwicklung zweckmäßig, wenn unmittelbar nach Aufnahme des ersten Bilddatensatzes automatisch durch Verstellen der Kompressionsplatte der zweite Kompressionsgrad eingestellt wird, wonach unmittelbar der zweite Bilddatensatz aufgenommen wird und anschließend, gegebenenfalls nach vorheriger Transformation, die Verknüpfung und danach auch die Ausgabe des ermittelten Bildes, beispielsweise des Differenzbildes, erfolgt. Die Änderung des Kompressionsgrads kann in beide Richtungen erfolgen, das heißt, die Mamma kann im Rahmen der Aufnahme des ersten Bildes stärker komprimiert und zur Aufnahme des zweiten Bildes entlastet werden, oder umgekehrt. Denn in jedem Fall kommt es zu einer elastischen Gewebebewegung, woraus die Gewinnung der elastographischen Informationen resultiert.

Neben dem Verfahren selbst betrifft die Erfindung ferner ein Mammographiegerät umfassend eine Strahlungsquelle, einen digitalen Strahlungsdetektor, eine Auflageplatte und eine Kompressionsplatte, zwischen denen die Mamma, die über die Kompressionsplatte komprimiert wird, aufgenommen wird, sowie eine Bildverarbeitungseinrichtung, ausgebildet zur Durchführung des beschriebenen Verfahrens.

Dokument US 2007/238966 A1 offenbart ein Verfahren, zur Simulation der Erzeugung von Gewebeeigenschaften, mit einem Mammographiegerät, umfassend eine Strahlungsquelle, einen Strahlungsdetektor, eine Auflageplatte und eine Kompressionsplatte, zwischen denen die Mamma, die über die Kompressionsplatte komprimiert wird, aufgenommen ist, mit folgenden Schritten: ₋ Aufnahme eines die Mamma abbildenden ersten Bilddatensatzes bei einem ersten Kompressionsgrad der Mamma, Einstellung eines zweiten Kompressionsgrads der Mamma und Aufnahme eines die Mamma abbildenden zweiten Bilddatensatzes und rechnerische Verknüpfung des ersten und des zweiten Bilddatensatzes zur Erzeugung der Gewebeeigenschaften.

Weitere Vorteile, Merkmale und Einzelheiten der Erfindung ergeben sich aus dem im Folgenden beschriebenen Ausführungsbeispiel sowie anhand der Zeichnungen. Dabei zeigen:
- Fig. 1: eine Ansicht eines Mammographiegeräts, und
- Fig. 2: eine Prinzipdarstellung des Mammographiegeräts aus Fig. 1 zur Erläuterung des Ablaufs des erfindungsgemäßen Verfahrens.

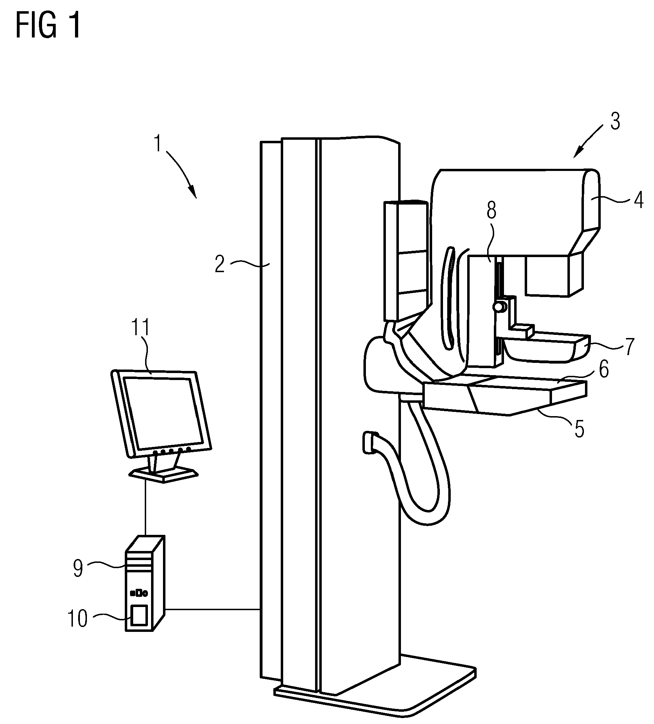

Fig. 1 zeigt ein an sich bekanntes Mammographiegerät 1, umfassend eine Vertikalsäule 2, an der vertikal und bewegbar die Bildaufnahmeeinheit 3 angeordnet ist. Diese umfasst eine Röntgenstrahlungsquelle 4 sowie einen digitalen Röntgenstrahlungsdetektor 5, der unterhalb einer Auflageplatte 6 für die weibliche Brust angeordnet ist. Oberhalb der Auflageplatte 6 ist eine Kompressionsplatte 7 vorgesehen, die ebenfalls an einem Vertikalträger 8 vertikal verstellbar geführt ist. Über diese Kompressionsplatte 7 kann die Brust in an sich bekannter Weise zur Bildaufnahme komprimiert werden. Vorgesehen ist ferner eine Steuerungseinrichtung 9, die den gesamten Betrieb des Röntgengeräts wie auch den Bildaufnahme- und Bildauswertebetrieb steuert, wozu eine geeignete Bildverarbeitungseinrichtung 10 vorgesehen ist. Aufgenommene Bilder werden an einem Monitor 11 ausgegeben.

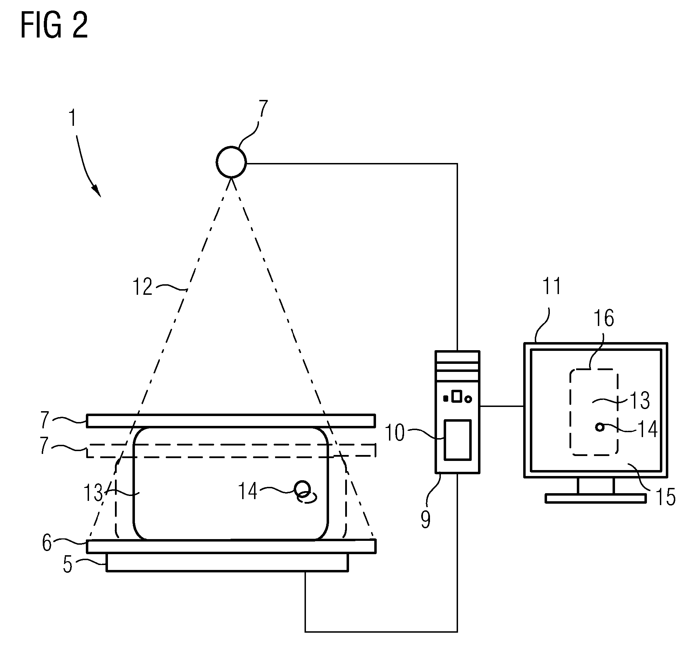

Fig. 2 zeigt in Form einer Prinzipdarstellung das Mammographiegerät 1 aus Fig. 1, um das erfindungsgemäße Verfahren näher zu erläutern. Gezeigt ist die Strahlungsquelle 4, die ein Röntgenstrahlungsbündel 12 emittiert. Dargestellt ist ferner die Auflageplatte 6 mit dem darunter befindlichen Strahlungsdetektor 5 sowie die Kompressionsplatte 7. Gezeigt ist weiterhin als Prinzipdarstellung die Mamma 13, die hier offensichtlich nicht in ihrer natürlichen Form dargestellt ist.

Die Mamma 13 enthält angenommener Maßen eine maligne Läsion 14, also beispielsweise ein Krebsgeschwür. Die Mamma 13 ist zwischen der Auflageplatte 6 und der Kompressionsplatte 7 gehalten und wird zwischen beiden komprimiert, der Kompressionsgrad kann durch den Grad der vertikalen Verschiebung der Kompressionsplatte 7 eingestellt werden.

Zu Beginn des Verfahrens befindet sich die Kompressionsplatte 7 in einer ersten Stellung, die hier durchgezogen dargestellt ist. Hierüber wurde ein erster Kompressionsgrad der Mamma 13 eingestellt. Im ersten Kompressionsgrad ist die Mamma 13 sowie die Läsion 14 ebenfalls durchgezogen dargestellt. Das Strahlungsbündel 12 durchstrahlt die Mamma 13 nebst Läsion 14, ein entsprechender Bilddatensatz, der die Ausgabe eines ersten Bildes ermöglicht, wird am Strahlungsdetektor 5 aufgenommen und in der Bildverarbeitungseinrichtung 10 der Steuerungseinrichtung 9 abgelegt.

Sodann wird im zweiten Verfahrensschritt die Kompressionsplatte 7 verstellt, im gezeigten Beispiel wird sie weiter zur Auflageplatte 6 hinbewegt, was dazu führt, dass der Druck auf die Mamma 13 und die Läsion 14 erhöht wird, die Mamma 13 wird also zwangsläufig stärker komprimiert. Diese zweite Position der Kompressionsplatte 7 ist gestrichelt gezeigt, auch der zweite Kompressionsgrad der Mamma 13 wie auch der Läsion 14 ist gestrichelt dargestellt. Diese Druckerhöhung beziehungsweise Verstärkung der Kompression führt nun dazu, dass die Mamma 13 verformt wird, das heißt, sie wird stärker zusammengedrückt und dehnt sich seitlich im Raum aus, wie Fig. 2 zeigt. Die Veränderung des Kompressionsgrads führt ebenfalls zu einer lateralen Verschiebung der Läsion 14. Die Verformung der Läsion 14 ausgehend von dem ersten Kompressionsgrad, wo sie also ausgezogen dargestellt ist, hin zur Lage wie sie gestrichelt dargestellt ist hängt stark von den elastischen Eigenschaften der Läsion ab. Diese sind für gutartige und maligne Läsionen deutlich unterschiedlich. Während eine gutartige Läsion ein elastisches Verhalten zeigt, das näherungsweise dem des gesunden Gewebes, das die Mamma 13 ansonsten umfasst, entspricht, ist eine maligne Läsion deutlich härter, sie verhält sich also in ihrer Ausweichbewegung bei einer Änderung des Kompressionsdrucks deutlich anders als eine gutartige Läsion. Handelt es sich also bei der Läsion 14 um eine gutartige Läsion, so würde sie an einer anderen Stelle liegen als gestrichelt dargestellt ist. Die gestrichelte Darstellung der Läsion 14 zeigt die Lage einer malignen Läsion an.

In jedem Fall wird nun auch in der zweiten Kompressionsstellung ein zweiter Bilddatensatz aufgenommen, die Bildsignale des Strahlungsdetektors 5 werden wiederum in der Bildverarbeitungseinrichtung 10 abgelegt.

Sodann wird der zweite Bilddatensatz rechnerisch derart transformiert, dass die durch die elastische Verformung auftretenden Größen- und Ortsveränderungen kompensiert werden. Es erfolgt also eine Rückrechnung der Bildsignale auf den Ausgangszustand, den die Mamma im Rahmen der ersten Bildaufnahme hatte. Das zweite Bild beziehungsweise der zweite Bilddatensatz wird also quasi auf den ersten Bilddatensatz transformiert. Dies erfolgt unter einer Annahme eines homogenen Elastizitätsverhaltens über die gesamte Fläche beziehungsweise das gesamte Volumen der Mamma 13, unabhängig davon, ob Läsionen vorhanden sind oder nicht. Hieraus resultiert aber auch, dass eine maligne Läsion 14 anders transformiert wird beziehungsweise quasi auf eine andere Ausgangslage bei Anliegen des ersten Kompensationsgrads zurückgerechnet wird, als sie tatsächlich im Volumen lag. Denn wie beschrieben ist das Kompressions- oder Elastizitätsverhalten einer malignen Läsion ein anderes als einer gutartigen Läsion, die ein näherungsweise dem gesunden Gewebe entsprechendes Verhalten aufweist.

Im nächsten Schritt wird eine rechnerische Verknüpfung des ersten, nicht transformierten Bilddatensatzes und des zweiten, transformierten Datensatzes vorgenommen, beispielsweise eine einfache Differenzbildung, gegebenenfalls unter Wichtung eines Bilddatensatzes. Beispielsweise wird das Differenzbild D zu D = A - 0,7 B berechnet, wobei A der erste Bilddatensatz und B der transformierte zweite Bilddatensatz ist. Es sind jedoch beliebige rechnerische Verknüpfungen mit beliebigen Wichtungen oder Berechnungsformeln möglich. Die Verarbeitung kann auch derart erfolgen, dass noch, wenngleich nicht allzu deutlich, die Umrisse der Mamma, vorzugsweise ausgehend vom nicht transformierten Bilddatensatz, im Differenzbild noch sichtbar sind, oder der Rand der Mamma, beispielsweise über einen Kantendetektionsalgorithmus ermittelt, etc. Dies deshalb, um dem Bediener die leichtere Orientierung im Differenzbild zu geben.

In jedem Fall wird das auf welche Weise auch immer erstellte Differenzbild respektive rechnerische Verknüpfungsbild dann am Monitor 11 ausgegeben, siehe Fig. 2, wo ein solches Differenzbild 15 dargestellt ist. Gezeigt ist zum einen gestrichelt der noch leicht sichtbare Rand 16 der Mamma 13 sowie die Läsion 14, die im Differenzbild deutlich hervortritt. Der Betrachter erhält also auf diese Weise ein deutlich spezifischeres Differenzbild, verglichen mit den einzelnen Bilddaten, die die beiden Bilddatensätze, die zu den unterschiedlichen Kompressionsgraden aufgenommen wurden, ergeben. In diesen wäre die Läsion 14 nur als leichter Schatten zu sehen, sie würde sich lange nicht so deutlich im Bild zeigen, wie dies aufgrund der erfindungsgemäßen Verknüpfung der Elastographie, also Ausnutzung der unterschiedlichen elastischen Eigenschaften des durchleuchteten Gewebes und der Durchleuchtungsmammographie nun möglich ist.

## Patentansprüche

1. Verfahren zur Erzeugung eines Bildes mit einem Mammographiegerät (1), umfassend eine Strahlungsquelle (4), einen digitalen Strahlungsdetektor (5), eine Auflageplatte (6) und eine Kompressionsplatte (7), zwischen denen die Mamma (13), die über die Kompressionsplatte (7) komprimiert wird, aufgenommen ist, **gekennzeichnet durch** folgende Schritte:
- Aufnahme eines die Mamma (13) abbildenden ersten Bilddatensatzes bei einem ersten Kompressionsgrad der Mamma (13), wobei die zwischen der Auflageplatte (6) und der Kompressionsplatte (7) gehaltene Mamma (13) zwischen diesen komprimiert wird,
- Einstellung eines zweiten Kompressionsgrads der Mamma (13) und Aufnahme eines die Mamma (13) abbildenden zweiten Bilddatensatzes,
- rechnerische Verknüpfung des ersten und des zweiten Bilddatensatzes zur Erzeugung des Bilds, wobei im Rahmen der Verknüpfung die beiden Bilddatensätze ungleich gewichtet werden.

2. Verfahren nach Anspruch 1, **dadurch gekennzeichnet, dass** einer der beiden Bilddatensätze vor der Verknüpfung unter Berücksichtigung eines homogenen elastischen Verhaltens der Mamma (13) derart transformiert wird, dass die aus der Veränderung des Kompressionsgrads resultierende elastische Veränderung der abgebildeten Mamma (13) kompensiert wird.

3. Verfahren nach Anspruch 1 oder 2, **dadurch gekennzeichnet, dass** im Rahmen der Verknüpfung ein Differenzbilddatensatz aus den beiden Bilddatensätzen und aus diesem ein Differenzbild (15) ermittelt wird.

4. Verfahren nach einem der vorangehenden Ansprüche, **dadurch gekennzeichnet, dass** die Bilddatensätze derart verknüpft werden, dass der Verlauf des Randes (16) der in einem der beiden Bilddatensätze, insbesondere in dem nicht transformierten Bilddatensatz, gezeigten Mamma (13) im Bild, gegebenenfalls dem Differenzbild (15) dargestellt wird.

5. Verfahren nach Anspruch 4, **dadurch gekennzeichnet, dass** der Rand (16) im einen Bilddatensatz mittels eines Kantendetektionsalgorithmus ermittelt und im Bild dargestellt wird.

6. Verfahren nach einem der vorangehenden Ansprüche, **dadurch gekennzeichnet, dass** unmittelbar nach Aufnahme des ersten Bilddatensatzes automatisch durch Verstellen der Kompressionsplatte (7) der zweite Kompressionsgrad eingestellt wird, wonach unmittelbar der zweite Bilddatensatz aufgenommen wird und anschließend, gegebenenfalls nach vorheriger Transformation, die Verknüpfung erfolgt.

7. Mammographiegerät umfassend eine Strahlungsquelle (4), einen digitalen Strahlungsdetektor (5), eine Auflageplatte (6) und eine Kompressionsplatte (7), zwischen denen die Mamma (13), die über die Kompressionsplatte (7) komprimiert wird, aufgenommen wird, sowie eine Bildverarbeitungseinrichtung (10), ausgebildet zur Durchführung des Verfahrens nach einem der vorangehenden Ansprüche.

## Claims

1. Method for generating an image using a mammography device (1) comprising a radiation source (4), a digital radiation detector (5), a support plate (6) and a compression plate (7), between which the breast (13) which is compressed by way of the compression plate (7) is accommodated, **characterized by** the following steps:
- recording a first image data record imaging the breast (13) at a first degree of compression of the breast (13), wherein the breast (13) held between the support plate (6) and the compression plate (7) is compressed between these,
- setting a second degree of compression of the breast (13) and recording a second image data record imaging the breast (13),
- linking the first and second image data record by computation in order to generate the image, wherein the two image data records are weighted unequally within the scope of linking.

2. Method according to Claim 1, **characterized in that**, prior to linking, one of the two image data records is transformed taking into account a homogeneous elastic behaviour of the breast (13) in such a way that the elastic change of the imaged breast (13) resulting from the change in the degree of compression is compensated.

3. Method according to Claim 1 or 2, **characterized in that**, within the scope of linking, a difference image data record is established from the two image data records and a difference image (15) is established therefrom.

4. Method according to one of the preceding claims, **characterized in that** the image data records are linked in such a way that the course of the edge (16) of the breast (13) shown in one of the two image data records, in particular in the non-transformed image data record, is depicted in the image, possibly in the difference image (15).

5. Method according to Claim 4, **characterized in that** the edge (16) is established in an image data record by means of an edge detection algorithm and depicted in the image.

6. Method according to one of the preceding claims, **characterized in that** the second degree of compression is set automatically by adjusting the compression plate (7) immediately after recording the first image data record, after which the second image data record is recorded immediately and linking is subsequently carried out, possibly after a preceding transformation.

7. Mammography device comprising a radiation source (4), a digital radiation detector (5), a support plate (6) and a compression plate (7), between which the breast (13) which is compressed by way of the compression plate (7) is accommodated, and an image processing apparatus (10) embodied to carry out the method according to one of the preceding claims.

## Revendications

1. Procédé de production d'une image avec un appareil de mammographie (1), comprenant une source de rayonnement (4), un détecteur numérique de rayonnement (5), une plaque d'appui (6) et une plaque de compression (7), entre lesquelles est accueilli le sein (13) qui est comprimé par l'intermédiaire de la plaque de compression (7), **caractérisé par** les étapes ci-dessous consistant à :
- enregistrer une première série de données d'image représentant le sein (13) pour un premier degré de compression du sein (13), dans lequel le sein (13) maintenu entre la plaque d'appui (6) et la plaque de compression (7) est comprimé entre ceux-ci,
- ajuster un deuxième degré de compression du sein (13) et enregistrer une deuxième série de données d'image représentant le sein (13),
- chaîner par calcul les première et deuxième séries de données d'image afin de produire l'image, dans lequel les deux séries de données d'image sont pondérées de manière différente dans le cadre dudit chaînage.

2. Procédé selon la revendication 1, **caractérisé en ce qu'**une des deux séries de données d'image est transformée avant ledit chaînage en prenant en compte un comportement élastique homogène du sein (13) de telle manière que la modification élastique, résultante de la modification du degré de compression, du sein (13) représenté est compensée.

3. Procédé selon la revendication 1 ou 2, **caractérisé en ce qu'**une série de données d'image différentielle est déterminée à partir des deux séries de données d'image, et une image différentielle (15) est déterminée à partir de ladite série.

4. Procédé selon l'une quelconque des revendications précédentes, **caractérisé en ce que** les séries données d'image sont chaînées de telle manière que le tracé du bord (16) du sein (13) montré dans une des deux séries de données d'image, en particulier dans la série de données d'image non transformée, est représenté sur l'image, éventuellement sur l'image différentielle (15).

5. Procédé selon la revendication 4, **caractérisé en ce que** le bord (16) est déterminé dans une série de données d'image au moyen d'un algorithme de détection de bordure et est représenté sur l'image.

6. Procédé selon l'une quelconque des revendications précédentes, **caractérisé en ce que** le deuxième degré de compression est ajusté de manière automatique par déplacement de la plaque de compression (7) immédiatement après enregistrement de la première série de données d'image, après quoi la deuxième série de données d'image est immédiatement enregistrée et le chaînage a ensuite lieu, éventuellement après une transformation préalable.

7. Appareil de mammographie comprenant une source de rayonnement (4), un détecteur numérique de rayonnement (5), une plaque d'appui (6) et une plaque de compression (7), entre lesquelles est accueilli le sein (13) qui est comprimé par l'intermédiaire de la plaque de compression (7), ainsi qu'un appareil de traitement d'image (10), conçu pour mettre en oeuvre le procédé selon l'une quelconque des revendications précédentes.
